# EUROPEAN PATENT APPLICATION

(11) **EP 2 532 355 A1**
(43) Date of publication of application: **12.12.2012**
(21) Application number: 11168839.6
(22) Date of filing: 06.06.2011
(51) Int. Cl.: A61K 36/28, A61P 25/14, A61P 25/16, A61P 25/28, A61P 25/08, A23L 1/00

(54) **Chicory for prevention and treatment of neurodegeneration**

(71) Applicant: Nestec S.A., 1800 Vevey (CH)
(72) Inventor: Durga, Jane, 1860 Aigle (CH); Kunz, Tina, 1012 Lausanne (CH); Yvon, Cédric, 1110 Morges (CH); Courtois, Didier, 37550 St-Avertin (FR); Husson, Jwanro, 37390 Notre Dame d'Oe (FR); Luthi-Carter, Ruth, 1028 Preverenges (CH); Taylor, David, Milton, Ontario L9T 5V9 (CA)
(74) Representative: Marquardt, Ulf

(57) **Abstract**

The invention pertains to a composition comprising chicory for use in the prevention or treatment of a neurodegenerative disorder and/or a loss of cognitive ability. In a further aspect, the invention pertains to a non-therapeutic method for maintaining mental or cognitive capabilities.

## Description

### Field of the invention:

The invention pertains to a composition comprising chicory for use in the prevention or treatment of a neurodegenerative disorder and/or a loss of cognitive ability. In a further aspect, the invention pertains to a non-therapeutic method for maintaining mental or cognitive capabilities.

### Background of the invention:

Neurodegenerative disorders are characterized by a progressive loss of structure and function of neurons, ultimately leading to death of neurons. In many diseases such as Alzheimer's, Parkinson's or Huntington's disease neurodegenerative processes are a major detrimental component, modulating the course of disease. The biggest risk factor for neurodegenerative diseases is aging. Many of these diseases are late-onset, meaning that there is some factoring that changes as a person gets older. One constant factor is that in each disease, neurons gradually lose function as the disease progresses with age. A further consequence of such continuous and severe loss of neuronal function is the loss of the cognitive ability as can be manifested in different forms of dementia. Thereby, normal cognitive functions can be affected with for example a loss of memory, attention or mental concentration, language, and the ability to solve problems. Especially in the later stages of a neurodegenerative condition, affected persons may be disoriented in time, in place, and in person. Neurodegenerative disorders, though often treatable to some degree, are usually due to causes that are progressive and incurable. There is a clear and persisting need in finding a solution for preventing and treating neurodegenerative disorders and the loss of cognitive abilities due to the progressive degeneration of neurons, particularly for the aging population.

### Summary of the invention:

The object of the present invention is to provide a natural solution to the above cited need which can be applied via medication or consumed as a food for preventing or treating such neurodegenerative disorders.

The object of the present invention is achieved by the subject matter of the independent claims. The dependent claims further develop the idea of the present invention.

Accordingly, the present invention provides in a first aspect a composition comprising chicory for use in the prevention or treatment of a neurodegenerative disorder and/or a loss of cognitive ability.

"Neurodegenerative disorders" are defined here as hereditary or sporadic conditions which are characterized by progressive nervous system dysfunction. These disorders are often associated with atrophy of the affected central or peripheral structures of the nervous system. They include diseases such as Alzheimer's disease and other dementias, degenerative nerve diseases, epilepsy, genetic brain disorders, Parkinson's disease, amyotrophic lateral sclerosis (ALS or Lou Gehrig's disease), Huntington's disease, and prion diseases.

"Cognitive ability" is defined here as the intellectual process by which an individual becomes aware of, perceives, or comprehends ideas. Cognitive ability embraces the quality of knowing, which includes all aspects of perception, recognition, conception, sensing, thinking, reasoning, remembering and imaging. Loss of cognitive ability is the difficulty in dealing with or reacting to new information or situations.

It has been surprisingly found by the inventors that chicory extracts robustly increase neuronal survival in an *in vitro* model of neuronal senescence. Therefore, chicory extracts may represent an attractive pharmaceutical and/or nutritional solution against neurodegeneration.

Before being tested *in vivo* for example in an animal study, compounds or compositions with potential neuroprotective activity are typically first tested *in vitro* in neuronal cell cultures. This also allows further mechanistic investigations as for example with transcriptomic and proteomic tools. The beneficial effect of such compounds or compositions on cell survival is classically investigated by challenging the cell cultures with neurotoxic insults using glutamate receptor agonists (e.g. glutamate, N-methyl-D-aspartate (NMDA) or kainic acid) or oxidants (e.g. hydrogen peroxide, H₂O₂) and measuring the resulting oxidative stress and excitotoxicity over a limited period of time, e.g. over a few days (Aksenova, M.V. et al., 2005, Curr. Neurovas. Res. 2, 73-89; Nicholls, D.G., 2004, Curr. Mol. Med. 4(2), 149-177). Cell survival is then compared between treated-cultures and non-treated cultures. However, challenging the cell cultures over such a limited period of time is most likely not correctly mimicking the slow degenerative processes that occur naturally in humans or animals. Hence, the inventors made use of a culture model where neurons were not challenged with a neurotoxic insult and thus undergo a natural progressive senescence over a much longer time period, e.g. over several weeks. This bioassay much better mimicks the actual process as observed *in vivo.*

Thereby, the inventors found that several different extracts of chicory significantly increased the survival of neuronal cells as compared to the DMSO control samples, when incubated for several weeks in a culture medium. Survival was evaluated using NeuN (Neuronal Nuclei) immune-staining (Mullen, R.J. et al., 1992, Develop. 116, 201-211; Wolf, H.K. et al., 1996, Cytochem. 44(10), 1167-1171). This significant effect of cell survival was further confirmed by looking at a molecular indicator of connectivity, namely PSD-95 (Colledge, M. et al., 2003, Neuron 40, 595-607) in the cell cultures, which was also markedly preserved as compared to the control samples.

The treatment with the chicory extract was further performed in parallel with a positive control sample. This control consisted of a synthetic compound inhibiting SIRT2 (AK-1), which was shown previously to protect against cell death in a model of Parkinson's disease (Outeiro, T.M. et al., 2007, Science 317, 516-519). Surprisingly, this positive control sample, although being significantly positive over non-treated cells, showed a smaller neuroprotective effect than the chicory extract in the improved cell culture bioassay. This may suggest that the beneficial effect of chicory is mediated by either a better inhibiting effect of SIRT2 or by a different mechanism that underlies a greater protection of the neuronal cells. For the time being, the mechanism of action of the chicory extracts to those cells is not known.

### Brief Description of the Drawings:

Figure 1: Cortical longevity model with death of neurons depicted from 4 weeks of age to (A) 6 weeks and (B) 8 weeks.
Figure 2: NeuN counts of cells at 8 weeks *in vitro* treated with an extract of roasted chicory root (Sample 1) showing neuroprotective effects at (A) 50 µg/ml and (B) 100 µg/ml.
Figure 3: NeuN counts of cells at 8 weeks *in vitro* treated with ethanol/water extracts of roasted chicory root (Sample 2; A and B) or with ethyl acetate extracts of dried chicory root (Sample 4; C) or roasted chicory root (Sample 3; D). The data show neuroprotective effects for the ethanol/water extract of roasted chicory root (Sample 2) at (A) 50 µg/ml and (B) 100 µg/ml. (C) The ethyl acetate extract of dried chicory root (Sample 4) showed neuroprotective effects at 50 µg/ml. (D) The roasted chicory roots (Sample 3) showed neuroprotective effects at 100 µg/ml.

### Detailed Description of the invention:

The present invention pertains to a composition comprising chicory for use in the prevention or treatment of a neurodegenerative disorder and/or a loss of cognitive ability, wherein the chicory is the root of a chicory plant. Preferably the root is dried or roasted. Dried or roasted chicory roots are an already existing industrial raw material. Hence, this has the advantages that chicory roots can be used in the most industrial available form, e.g. as dried roots or roasted as industrially provided for the production for specific beverages. Furthermore, dried chicory roots and even more preferably roasted chicory roots proved to be an excellent raw material for obtaining active compositions for use in the prevention or treatment of neurodegenerative disorders or a loss of cognitive ability.

In a preferred embodiment, the chicory is provided in the form of an extract. This allows on one hand to first concentrate the active component of the chicory for e.g. more effective and concentrated dosing, and on the other hand also to standardize and better control samples and their effective activity in for example batch wise production mode.

In one embodiment, the extract is obtainable from an ethanol-water extraction of the chicory root. Thereby, the chicory roots are first cut in slices or dices, and dried. Thereafter, they are extracted directly or after roasting with a mixture of ethanol and water. After centrifugation or filtration, the liquid is evaporated to give the extract. The advantage of using ethanol and water for the extraction process is that a crude extract can be obtained which is food-grade. The obtainable extract is soluble in water or in an ethanol/water mixture.

In another embodiment, the extract is obtainable from an ethyl-acetate extraction of the chicory root. Thereby, after grinding of the slices or dices of the dried or roasted chicory roots, the resulting powder is extracted by hexane to remove lipids and then dried. This dried powder without lipids is treated with a boiling solution of hydrochloric acid which is further extracted with ethyl-acetate. The organic phase is separated and evaporated to give the ethyl-acetate extracts. The advantage of the ethyl-acetate extraction is that the obtained extracts are much more concentrated. The ethyl-acetate extraction is much more selective and allows better enriching of the active principles. However, the resulting extract is usually not soluble in water and can in this form not be considered for being used directly e.g. in a food product.

In one embodiment, the composition of the invention is for use in the prevention or treatment of a neurodegenerative disorder, wherein the neurodegenerative disorder is Alzheimer's disease, Parkinson's disease, Huntington's disease, dementia or epilepsy. Acute and chronic neurological disorders, including Alzheimer's disease (AD), epilepsy, trauma, stroke and Parkinson's disease (PD), are characterized by neurodegeneration often resulting in neurological deficits. The neurological deficits can be so severe that the patient's ability to cope with everyday life is largely impaired, which consequently reduces the patient's quality of life. Therefore, effort has focused to identify strategies to prevent, delay or slow-down neurodegeneration and as a result cognitive decline. To identify substances which can modulate or stop cell death pathways and thus save cells from neurodegeneration has been proven difficult. Targeting healthy or damaged neurons with the invention can improve the ability of the neurons to cope with or even withstand neurotoxic insults (e.g. oxidative stress) and as a result improve neuronal survival. This improvement in neuronal survival and the correlated reduction in neurodegeneration is likely to also modulate the course of the neurological deficits.

In a further embodiment, the composition of the invention is for use in the prevention or treatment of a loss of cognitive ability, wherein the loss of cognitive ability is a loss of memory, attention, language, ability to reason and/or a cognitive dysfunction.

The composition of the invention may be a medicament, a food product or a supplement to a food product.

In one embodiment, the composition of the invention is intended for consumption by a human, preferably an adult human being. Many of the neurodegenerative disorders or cognitive dysfunctions occur with the progression of age of an individual. Clinical manifestation is therefore often only perceived in adulthood or at an already advanced age. Hence, the invention is preferably intended for adult persons, while or before the onset of such a neurodegenerative disorder or loss of cognitive ability. Thereby, advantageously, the disorder or cognitive dysfunction is treated early on to limit or reduce the further progression of the degeneration of neuronal cells. Ideally even, the onset of such degeneration can be delayed or reduced due to a preventive effect of an early application in adulthood, when the individual is still healthy and in his full cognitive capacity.

In an alternative embodiment, the composition of the invention is intended for consumption by an animal, preferably a cat or a dog. Similarly as with humans, neurodegeneration can be observed with animals, in particular with farm animals and animals kept as pets. Thereby, it is particularly difficult and heart breaking for owners of a cat or a dog to see their dear companion animal being affected by a neurodegenerative disorder or a loss of cognitive ability with the progression of the age of the animal. Advantageously, the current invention provides a solution which can also be provided to a companion animal by his owner.

The composition of the invention is preferably intended for a consumption regime over an extended period of time, preferably over several years. Neurodegenerative disorders and loss of cognitive ability are slow processes, which can occur only gradually, but progressively over many years and ultimately may lead to the death of an affected individual. Typically, persons affected with such a degenerative disorder, depending on the nature of which disease, may be affected and survive for 5, 10, 15, 20 years or longer. Advantageously, therefore, the composition of the invention is used for the entire such period or preferably even already from starting before the onset of such a disorder by an individual, in order to be most effective.

A further aspect of the present invention is a non-therapeutic method for maintaining mental or cognitive capabilities of an individual, the method comprising administering to said individual a composition comprising chicory. The advantage of the non-therapeutic method of the present invention is for healthy individuals, who are not diagnosed or at immediate risk for a neurodegenerative disorder. Thereby, healthy individuals can prevent or stop already very minor neurodegenerative effects before manifestation of any disease or disorder state. Thereby, mental or cognitive capabilities of those individuals can be maintained or even improved.

In a specific embodiment, the non-therapeutic method of the invention pertains to the mental or cognitive capabilities that are selected from the group consisting of mental concentration, sustained attention, retentive memory, mental alertness, ability to learn, executive functions, ability to reason, mood, and resistance to stress.

Those skilled in the art will understand that they can freely combine all features of the present invention disclosed herein. In particular, features described for the composition of the present invention may be combined with the non-therapeutic method of the present invention and vice versa. Further, features described for the different embodiments of the present invention may be combined. Further advantages and features of the present invention are apparent from the figures and examples.

### Examples:

### Example 1: Preparation of an ethanol/water extract from roasted chicory roots.

Chicory roots were cut in slices and were roasted at an industrial scale at 160°C for 80 minutes. After roasting, the roots were ground into a powder (0.5mm sieving) and extracted with an ethanol/water (50/50) mixture (2.5 L of ethanol/water solution per 500g of chicory root powder) for 2h under agitation. After centrifugation at 6000rpm for 10min at 20°C, the ethanol was evaporated (with a Rotavapor at 46°C). The remaining water phase was freeze-dried to result in the extract. The yield of the extraction was: 585g extract per 1kg of initial chicory powder.

### Example 2: Preparation of an ethyl-acetate extract from roasted chicory roots.

Chicory roots were cut in slices and were roasted as in Example 1. After roasting, the roots were ground into a powder (0.5mm sieving) and extracted 3 times with hexane for 1h each. The hexane phases were discarded and the ground was dried at room temperature overnight. The dried ground was then hydrolyzed with a 1N HCl solution for 1h30 at 100°C (850ml HCL 1N for 100g powder). After 1h30, the acidic aqueous fraction was extracted 3 times with 500ml ethyl-acetate. The three ethyl-acetate phases were joined, washed with water to reduce the acidity and then evaporated to give the ethyl-acetate fraction. The yield was: 76g extract per 1kg initial chicory powder.

### Example 3: Primary cortical neuron aging model.

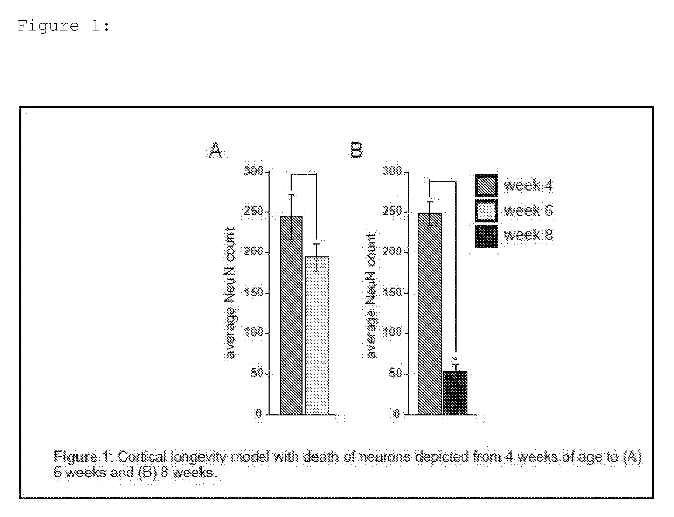

A primary cortical culture system to study the effects of compounds on neuronal aging has been developed and applied to study the effects of chicory on neuronal survival. This cell culture system has been developed as a model of neuronal aging in order to mimic processes which occur during normal aging of neurons without a neurotoxic challenge. With this model beneficial effects against physiological neurodegeneration during aging can be studied and compounds which increase neuronal survival can be identified. This is a different approach from the models described in the prior art in which the cells are challenged with a neurotoxic insult in order to induce different degrees of neurodegeneration. Using immune-cytochemical analysis of the antigen, NeuN (Neuronal Nuclei), which is a sensitive marker of neurodegeneration, a reliable method for examining the lifespan of cortical neurons in culture has been established. The sensitivity of NeuN can be attributed to its steady reduction in expression intensity as neurons degenerate, which is in contrast to many antigens whose expression can distinguish only between living and dead neurons. Between four and six weeks after plating, the cortical neurons begin to degenerate, leading to a non-significant trend in NeuN count over this time (Figure 1A). By week 8, the reduction in NeuN count is significant as compared to week 4 (Figure 1B), indicating that there is a gradual loss of neurons over several weeks, providing a chronic window to screen for nutritional compounds with the ability to decrease the slope of this neuronal senescence. All tests were fixed at a time point between 6 and 8 weeks.

### Primary culture preparation:

Striatal and cortical cultures from E16 rats were prepared according to a previously described procedure (Zala et al., 2005, Neurobiol. Dis. 20(3), 785-798). Pregnant Sprague-Dawley rats (Charles River Laboratories, France) were sacrificed by CO₂ inhalation. Embryos were collected on ice and dissected in medium containing Ca²⁺― and Mg²⁺-free phosphate buffered saline, 0.6% D-glucose, 1% Pen-Strep (10,000 U/ml) and 10 mM HEPES (Invitrogen AG, Switzerland) using a stereomicroscope. Separated brain regions were minced with forceps, dissection medium was removed and tissue homogenized by repeated pipetting in DMX containing 1% bovine serum albumin (Fluka, Switzerland). Following centrifugation (4°C, 5 min, 1000Xg) and resuspension in Neurobasal™ medium (Invitrogen AG, Switzerland), cells were plated at a density of 50000 per well in 96-well dishes (COSTAR®, Corning Incorporate, USA) coated with 0.1 mg/ml poly-Llysine (MW: 30000-70000, Sigma, Switzerland). Cells were incubated at 37°C/5% CO in Neurobasal™ medium supplemented with 1X B―27 Supplement, 1X Pen/Strep, 500 µM L-glutamine and 15mM KCl until the analysis of neuronal survival at 8 weeks.

### NeuN immunostaining:

Neurons were fixed (at 6-8 weeks *in vitro)* with 4% paraformaldehyde/PBS (4°C, 10 min) then rinsed three times with 1XPBS. Blocking solution containing PBS with 10% normal goat serum and 0.1% Triton-X was applied to neurons (RT, 1 h, mild shaking) then cells were incubated with anti-NeuN (1:400; Chemicon, Germany) in PBS containing 5% normal goat serum and 0.1 % Triton-X (4°C, overnight, gentle rocking). Neurons were then rinsed three times with 1XPBS before incubation with anti-mouse Cy™3-conjugated AffiniPure F(ab')2 Fragment secondary antibody (1:800; Jackson ImmunoResearch Laboratories Inc., UK) covered with aluminium foil (RT, 2 h, mild shaking). Cultures were then rinsed three times with 1X PBS and protected from light for the duration of time prior to analysis. PSD-95 labeling was identical except that fixation was in 100% methanol (-20°C, 10 min). Anti-PSD-95 (ABR-Affinity Bioreagents) was used at a concentration of 1:500. Evaluation of NeuN cell count was performed using a BD Pathway™ 855 High-Content Bioimager (BD Biosciences, Belgium). Automated pictures of each well were made using identical parameters for unbiased comparison between conditions. Pictures were analyzed using Image J software by converting all images to a stack using a built-in image enhancement plugin and analyzing the number of fluorescent entities that satisfy rigid size (0.0014-0.028 square pixels) and circularity (0.5-1.0) requirements for consideration as a neuronal nucleus. PSD-95 images used for integrated pixel density measurements were acquired in an identical manner to NeuN. Statistical significance was assessed between data pairs with a one-tailed t-test using Microsoft® Excel. Significance was attributed using an α-level beginning at 0.05. P values, represented an asterisk, indicated as * ― p < 0.05.

### Example 4:̅ Activity of the chicory extracts (of Example 1) on the neuron aging model (of Example 3).

The chicory extracts described above demonstrated significant neuroprotection in an in vitro cortical culture model of aging. The cells were treated according to a standard regimen. Half the volume of the culture medium was replaced with normal medium on Day 11 and treatment was initiated on Day 17 by replacing half the medium with either the vehicle DMSO (controls) or with the required amount of one of the extracts to reach the desired final concentration. Half of the medium was replaced each week to maintain the viability of the cells using a medium that contained equivalent amounts of vehicle or extracts to reach the desired final concentrations. All treatment conditions were compared to cultures treated with the equivalent concentration of DMSO or water vehicle. Cell viability was evaluated using NeuN counts as described above. With NeuN only cell viability can be evaluated, therefore in order to correlate the effects of the extracts also on neuronal function another marker, namely PSD-95, which is an indicator of neuronal connectivity, has been used. This secondary readout gives a further measure of neuronal health. PSD-95 was validated for its relationship to neuronal firing behavior by correlating decreases in PSD-95-positive synapses with decreased burst firing activity in an *in vitro* cortical neuronal model of polyglutamine disease. Therefore, we confirm that this assay comprises an interesting and relevant secondary test for neuronal function in our primary cultures.

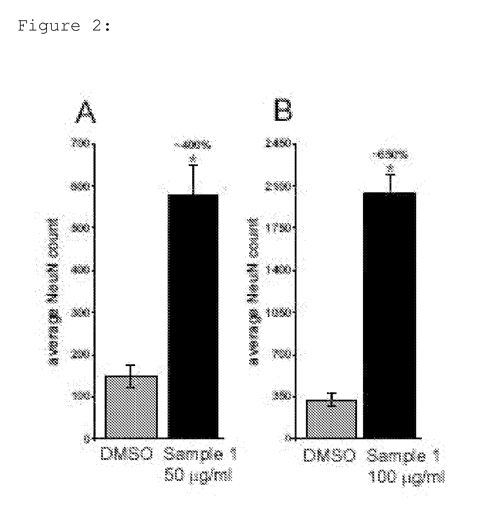

An extract of roasted chicory root (Sample 1) produced a significantly higher NeuN count than the corresponding DMSO control at both 50 µg/ml (400%, Figure 2A) and 100 µg/ml (650%, Figure 2B).

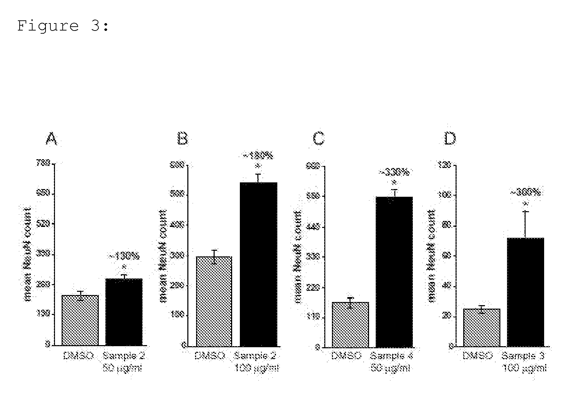

Testing of other chicory extracts confirmed neuroprotective effects of chicory extracts (Figure 3). A water/ethanol extract of roasted chicory roots (Sample 2) was neuroprotective at both 50 µg/ml (~130%, Figure 3A) and 100 µg/ml (~170-180%, Figure 3B). An extract of roasted chicory prepared by ethyl-acetate extraction after acidic hydrolysis (Sample 3) also demonstrated neuroprotection at 100 µg/ml (~300%, Figure 3D). Another extract from dried roots (Sample 4) but prepared by acidic hydrolysis, followed by methanol and ethyl-acetate extraction was strongly neuroprotective at 50 µg/ml (∼330%, Figure 3C).

## Claims

1. Composition comprising chicory for use in the prevention or treatment of a neurodegenerative disorder and/or a loss of cognitive ability.

2. The composition of claim 1, wherein the chicory is the root of a chicory plant.

3. The composition of claim 2, wherein the root is dried or roasted.

4. The composition of one of the preceding claims, wherein the chicory is provided in the form of an extract.

5. The composition of claim 4, wherein the extract is obtainable from an ethyl-acetate or ethanol/water extraction of the chicory.

6. The composition of one of the preceding claims, wherein the neurodegenerative disorder is Alzheimer's disease, Parkinson's disease, Huntington's disease, dementia or epilepsy.

7. The composition of one of the preceding claims, wherein the loss of cognitive ability is a loss of memory, attention, language, ability to reason and/or a cognitive dysfunction.

8. The composition of one of the preceding claims, which is a medicament, a food product or a supplement to a food product.

9. The composition of one of the preceding claims, which is intended for consumption by a human, preferably an adult human being.

10. The composition of one of the claims 1 to 8, which is intended for consumption by an animal, preferably a cat or a dog.

11. The composition of one of the preceding claims, which is intended for a consumption regime over an extended period of time, preferably over several years.

12. A non-therapeutic method for maintaining mental or cognitive capabilities of an individual, the method comprising administering to said individual a composition comprising chicory.

13. The non-therapeutic method of claim 12, wherein the mental or cognitive capabilities are selected from the group consisting of mental concentration, sustained attention, retentive memory, mental alertness, ability to learn, executive functions, ability to reason, mood, and resistance to stress.
